# EUROPÄISCHE PATENTSCHRIFT

(11) **EP 1 493 462 B1**
(45) Veröffentlichungstag und Bekanntmachung des Hinweises auf die Patenterteilung: **24.07.2019**
(21) Anmeldenummer: 04022931.2
(22) Anmeldetag: 12.11.2001
(51) Int. Cl.: A61M 16/06

(54) **Atemmaske mit Stirnstütze**
Respiratory mask with forehead support
Masque respiratoire muni d'un support frontal

(30) Priorität: 14.11.2000 DE 10056331
(43) Veröffentlichungstag der Anmeldung: 05.01.2005
(62) Teilanmeldung aus: 01126897.6
(73) Patentinhaber: Löwenstein Medical Technology S.A., 2557 Luxembourg (LU)
(72) Erfinder: Eifler, Martin, 25348 Glückstadt (DE); Schulz, Gerd, 22869 Schenefeld (DE)
(74) Vertreter: Klickow & Wetzel PartGmbB

(56) Entgegenhaltungen:
- WO-A-00/57942
- DE-U- 29 923 126
- US-A- 2 245 658
- US-A- 2 664 084
- US-A- 5 042 478
- US-A- 6 119 693

## Beschreibung

Die Erfindung betrifft eine Atemmaske mit einer Vorrichtung zur Abstützung der Atemmaske im Bereich einer Stirn eines Patienten, die mindestens ein im Bereich mindestens eines Halterungsschenkels der Atemmaske angeordnetes und über eine Verstelleinrichtung mit dem Halterungsschenkel verbundenes Stützelement aufweist, sowie bei der der Halterungsschenkel eine Längsachse aufweist sowie relativ zu einem Maskengrundkörper der Atemmaske verschwenkbar angeordnet ist, und bei der das Stützelement relativ zur Verstelleinrichtung unbeweglich angeordnet ist.

Derartige Stützelemente mit Verstelleinrichtung dienen dazu, eine an eine jeweilige Gesichtsgeometrie des Patienten angepaßte Abstützung einer Atemmaske im Bereich einer Stirn des Patienten vorzunehmen. Die bekannten Stirnstützen weisen entweder eine Verstellmöglichkeit auf, um eine Verstellung im wesentlichen senkrecht zur Stirn vorzunehmen und hierdurch den wirksamen Stirnabstand zu verändern, oder um eine Verstellung im wesentlichen parallel zur Gesichtsfläche vorzunehmen und hierdurch eine Anpassung an die jeweilige Stirnhöhe durchzuführen. Bekannt sind hierzu mechanische Verstellungen oder der gezielte Einbau von Elementen mit einer relativ zueinander unterschiedlichen geeigneten Dimensionierung.

Eine Stirnstütze mit einer Verstelleinrichtung zur Vorgabe eines wirksamen Stirnabstandes ist beispielsweise in der US-PS 61 19 693 beschrieben. Es wird hier eine brückenartige Stirnstütze offenbart, die über zwei Tragarme mit der Atemmaske verbunden ist. Ein Querträger des Stützelementes, der zwei Stirnpolster trägt, weist im Bereich seiner den Stützarmen zugewandten Ausdehnung Rastungen auf. Durch eine Arretierung der Stützarme in unterschiedlichen Rastungen wird ein unterschiedlicher Abstand des Querträgers zur Atemmaske und damit ein unterschiedlich wirksamer Stirnabstand vorgegeben.

Aus der US-A-2,245,658 ist bereits eine Vorrichtung zur Abstützung einer Atemmaske bekannt, die mit einem Stützelement versehen ist, das zur Anlage im Bereich einer Stirn eines Benutzers ausgebildet ist. Das Stützelement ist über eine Verstelleinrichtung und einen Halterungsschenkel mit der Atemmaske verbunden. Der Halterungsschenkel ist relativ zu einem Maskengrundkörper der Atemmaske verschwenkbar angeordnet. Das Stützelement und die Verstelleinrichtung sind einteilig ausgebildet.

Aus der US-A-5,042,478 ist eine weitere Vorrichtung zur Abstützung einer Beatmungsvorrichtung bekannt, die ein Stützelement aufweist, das mit einem Halterungsschenkel und einer Verstelleinrichtung gekoppelt ist. auch hier ist der Halterungsschenkel relativ zu einem Grundkörper verschwenkbar angeordnet. Das Stützelement ist einteilig mit der Verstelleinrichtung konstruiert.

Die bislang bekannten Vorrichtungen können im Hinblick auf ihren Einstellkomfort und ihre Bedienbarkeit noch nicht alle Anforderungen erfüllen, die aus einer Verwendung durch den Patienten selbst resultieren.

Aufgabe der vorliegenden Erfindung ist es daher, eine Vorrichtung der einleitend genannten Art derart zu konstruieren, daß eine vielseitige Einstellbarkeit bei gleichzeitig einfacher Bedienung bereitgestellt wird.

Diese Aufgabe wird erfindungsgemäß dadurch gelöst, daß die Verstelleinrichtung eine Querführung für die Positionierung des Stützelementes im Wesentlichen quer zur Längsachse des Halterungsschenkels aufweist, wobei für das Stützelement zur Fixierung einer Adaption an einen jeweiligen Stirnabstand eine lösbare Arretierung quer zur Längsachse vorgesehen ist.

Zur Fixierung einer Adaption an eine jeweilige Stirnhöhe wird vorgeschlagen, dass die Verstelleinrichtung eine Längsführung für das Stützelement in Richtung der Längsachse des Halterungsschenkels aufweist, und daß für die Verstelleinrichtung eine Arretierung in Richtung der Längsachse vorgesehen ist.

Zur Erleichterung einer Einstellung ist vorgesehen, daß für die Führung der Verstelleinrichtung in Richtung der Längsachse eine Rastung vorgesehen ist.

Darüber hinaus ist auch daran gedacht, daß die Verstelleinrichtung in Richtung der Längsachse stufenlos verstellbar ist.

Eine weitere Bedienungserleichterung kann dadurch erreicht werden, daß für die Verstellung des Stützelementes quer zur Längsachse eine Rastung vorgesehen ist.

Auch im Hinblick auf diese Verstellmöglichkeit ist daran gedacht, daß das Stützelement quer zur Längsachse stufenlos verstellbar ist.

Eine weiche Abpolsterung der Stirn kann dadurch erfolgen, daß das Stützelement ein Stirnpolster trägt, das als ein Gelkissen ausgebildet ist.

Darüber hinaus ist daran gedacht, daß das Stützelement ein Stirnpolster trägt, das als ein elastomeres Polster ausgebildet ist.

Ebenfalls ist es möglich, daß das Stützelement ein Stirnpolster trägt, das als ein Schaumpolster ausgebildet ist.

Eine weitere Erhöhung der Einstellungsflexibilität kann dadurch erreicht werden, daß das Stützelement relativ zum Halterungsschenkel verschwenkbar angeordnet ist.

Eine weitere Anpassung an einen individuellen Patienten kann dadurch erreicht werden, daß das Stirnpolster in frontaler Richtung verkippbar angeordnet ist.

Ein weiter gesteigerter Benutzungskomfort kann dadurch erreicht werden, daß das Stirnpolster seitlich kippbar angeordnet ist.

Eine besonders gute Anpassung an eine Stirnkontur wird dadurch unterstützt, daß das Stirnpolster als ein elastomeres Band ausgebildet ist, das von einer gabelartigen Halterung positioniert ist.

Gemäß einer weiteren Ausführungsvariante ist daran gedacht, daß das Stirnpolster als ein elastomeres Band ausgebildet ist, das einteilig mit einer gabelartigen Halterung konstruiert ist.

In den Zeichnungen sind Beispiele schematisch dargestellt, die das Verständnis der vorliegenden Erfindung erleichtern. Einige Zeichnungen zeigen eine Atemmaske mit einem Halterungsschenkel, welcher an einem Maskengrundkörper der Atemmaske befestigt (angeformt) ist. Erfindungsgemäss ist aber der Halterungsschenkel relativ zum Maskengrundkörper der Atemmaske verschwenkbar angeordnet.

Es zeigen:
- Fig. 1: Eine perspektivische Darstellung einer Atemmaske mit einem Stützelement, das sowohl in Richtung einer Längsachse eines Halterungsschenkels als auch quer zur Längsachse positionierbar ist,
- Fig. 2: eine Ansicht gemäß Blickrichtung II in Fig. 1,
- Fig. 3: eine Ansicht gemäß Blickrichtung III in Fig. 1,
- Fig. 4: eine perspektivische Darstellung gemäß Blickrichtung IV in Fig. 3,
- Fig. 5: eine Explosionsdarstellung des Stützelementes mit Stirnpolster und Verstelleinrichtung,
- Fig. 6: eine Darstellung der Anordnung gemäß Blickrichtung VI in Fig. 5,
- Fig. 7: eine vergrößerte perspektivische Darstellung des Stützelementes,
- Fig. 8: eine Seitenansicht des Stützelementes gemäß Blickrichtung VIII in Fig. 7,
- Fig. 9: eine vergrößerte perspektivische Darstellung des Stirnpolsters,
- Fig. 10: eine vergrößerte perspektivische Darstellung der Verstelleinrichtung,
- Fig. 11: eine Darstellung der verstelleinrichtung gemäß Blickrichtung XI in Fig. 10,
- Fig. 12: die Verstellvorrichtung gemäß Blickrichtung XII in Fig. 10,
- Fig. 13: eine Seitenansicht gemäß Blickrichtung XIII in Fig. 1,
- Fig. 14: eine weitere perspektivische Darstellung der Verstelleinrichtung,
- Fig. 15: einen Längsschnitt gemäß Schnittlinie XV-XV in Fig. 11 nach einer Drehung um 90°,
- Fig. 16: eine perspektivische Darstellung der Verstelleinrichtung gemäß Blickrichtung XVI in Fig. 14,
- Fig. 17: eine weitere perspektivische Darstellung der Verstelleinrichtung,
- Fig. 18: eine weitere perspektivische Darstellung des Stützelementes,
- Fig. 19: eine weitere perspektivische Darstellung des Stirnpolsters,
- Fig. 20: eine perspektivische Darstellung gemäß Blickrichtung XX in Fig. 19 nach einer Drehung um 90°,
- Fig. 21: eine Ansicht gemäß Blickrichtung XXI in Fig. 19,
- Fig. 22: einen Querschnitt gemäß Schnittlinie XXII-XXII in Fig. 19,
- Fig. 23: eine Prinzipdarstellung einer brückenartigen Stirnstütze und
- Fig. 24: eine gegenüber Fig. 23 abgewandelte Konstruktion einer brückenförmigen Stirnstütze in einteiliger Ausführung.

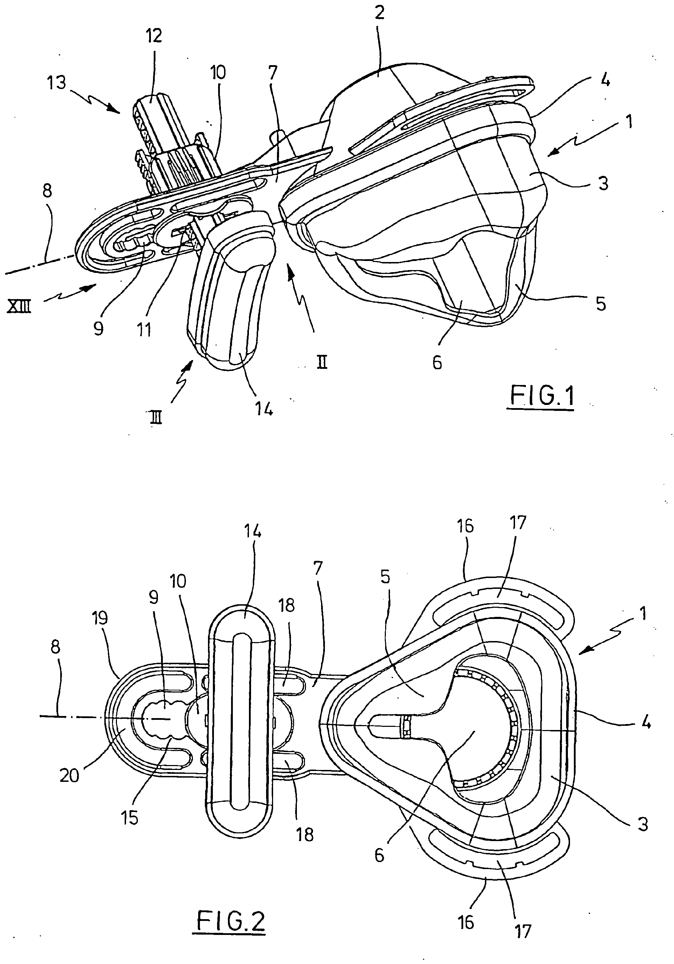

Fig. 1 zeigt eine perspektivische Darstellung einer Atemmaske (1), die im wesentlichen aus einem Maskengrundkörper (2) aus einem festen Material und einem Maskenpolster (3) aus einem weichen Material besteht. Das Maskenpolster (3) kann über einen Polstersockel (4) auf einen Rand des Maskengrundkörpers (2) aufgesteckt sein. Das Maskenpolster (3) begrenzt mit Dichtungslippen (5) eine Atemöffnung (6), in die bei einer Benutzung durch einen Patienten eine Nase des Patienten eingeführt wird.

Am Maskengrundkörper (2) ist ein Halterungsschenkel (7) befestigt, der sich mit einer Längsachse (8) im wesentlichen parallel zu einer von der Atemöffnung (6) aufgespannten Ebene erstreckt. Der Halterungsschenkel (7) weist eine Montageöffnung (9) auf, in die eine Verstelleinrichtung (10) eingesetzt ist. Die Verstelleinrichtung (10) weist quer zur Längsachse (8) eine Führungsausnehmung (11) auf, in die ein Schaft (12) eines Stützelementes (13) eingeführt ist. Das Stützelement (13) trägt ein Stirnpolster (14).

Aus der Darstellung in Fig. 2 ist erkennbar, daß sich das Stirnpolster (14) im wesentlichen quer zur Längsachse (8) erstreckt. Ebenfalls ist zu erkennen, daß die Montageöffnung (9) ähnlich zu einem Längsschlitz ausgebildet ist, der sich in Richtung der Längsachse (8) erstreckt. Gemäß der Ausführungsform in Fig. 1 und Fig. 2 weist die Montageöffnung (9) mehrere Rastungen (15) auf, um eine stufenweise definierte Positionierung der Verstelleinrichtung (10) in Richtung der Längsachse (8) zu unterstützen. Fig. 2 veranschaulicht ebenfalls, daß am Maskengrundkörper (2) seitlich zwei Flansche (16) angeformt sind, die jeweils mit schlitzförmigen Ausnehmungen (17) versehen sind.

Alternativ zu einer Verwendung von Rastungen (15) ist es auch denkbar, für die Positionierung der Verstelleinrichtung (10) in Richtung der Längsachse (8) mehrere Aufnahmen im Bereich des Halterungsschenkels (7) anzuordnen und die Verstelleinrichtung (10) jeweils in eine dieser Aufnahmen einzusetzen.

Im Bereich des Halterungsschenkels (7) sind seitlich zwei Ausnehmungen (18) und im Bereich eines Endsegmentes (19) ist eine hufeisenartige Ausnehmung (20) angeordnet.

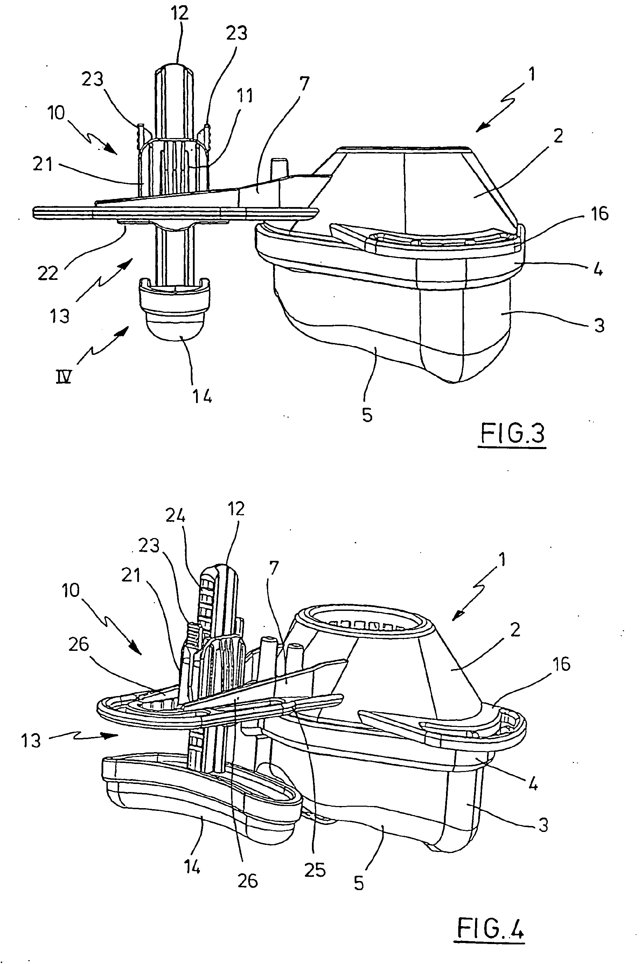

Aus Fig. 3 ist erkennbar, daß die Verstelleinrichtung (10) mit einem Grundkörper (21) durch die Montageöffnung (9) hindurchragt und mit einer Platte (22) auf dem Halterungsschenkel (7) aufliegt. Der Grundkörper (21) weist federnd gelagerte Klemmstege (23) auf, die den Schaft (12) des Stützelementes (13) innerhalb der Führungsausnehmung (11) der Verstelleinrichtung (10) fixieren.

Die perspektivische Darstellung in Fig. 4 veranschaulicht, daß der Schaft (12) im Bereich seiner den Klemmstegen (23) zugewandten Seiten mit einer Rastung (24) versehen ist, um auch hier eine definierte und schrittweise Positionierung des Stützelementes (13) zu ermöglichen.

Fig. 4 veranschaulicht ebenfalls, daß der Halterungsschenkel (7) im wesentlichen aus einer Schenkelplatte (25) sowie Stützstegen (26) besteht, die im wesentlichen senkrecht zur Schenkelplatte (25) angeordnet sind und zu einer erhöhten Biegesteifigkeit beitragen. Die Stützstege (26) verjüngen sich in eine dem Maskengrundkörper (2) abgewandte Richtung und sind im Bereich ihrer verdickten Enden am Maskengrundkörper (2) angeformt.

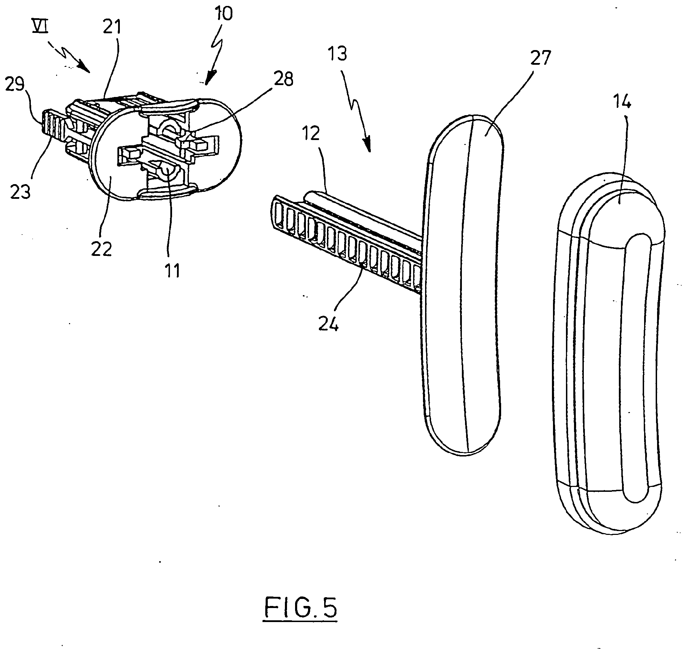

Fig. 5 veranschaulicht in einer Explosionsdarstellung die Konstruktion der Verstelleinrichtung (10) und des Stützelementes (13). Insbesondere ist erkennbar, daß das Stützelement (13) eine Stützplatte (27) aufweist, auf die das Stirnpolster (14) aufgesteckt wird. Ebenfalls ist aus Fig. 5 zu erkennen, daß die Klemmstege (23) mit Rastvorsprüngen (28) versehen sind, die in die Rastungen (24) eingreifen können. Bei einem manuellen Druck auf Bedienflächen (29) der Klemmstege (23) werden die Rastvorsprünge (28) aus der Rastung (24) herausgeführt.

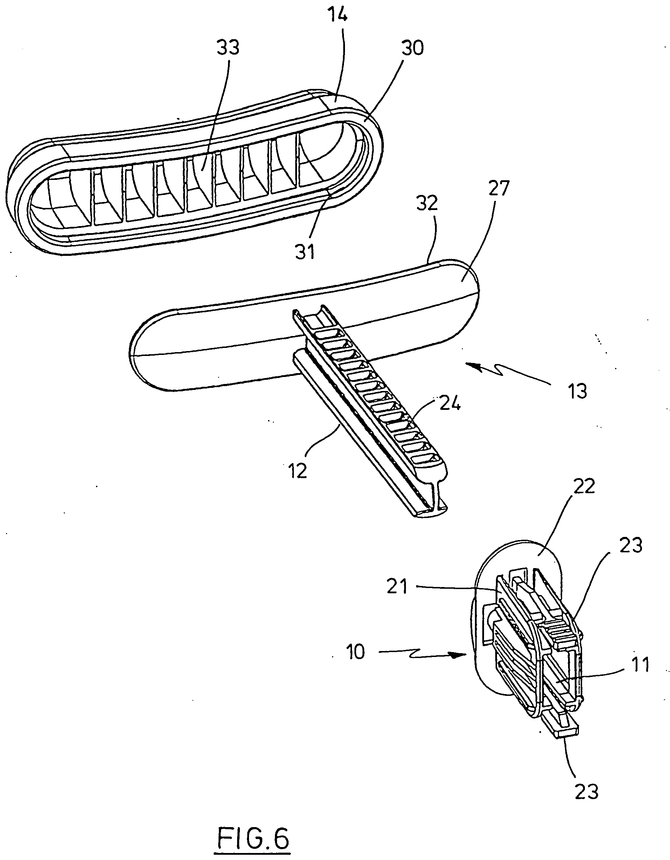

Fig. 6 veranschaulicht, daß das Stirnpolster (14) im Bereich eines Randes (30) mit einem U-Profil (31) versehen ist, das auf einen Rand (32) der Stützplatte (27) aufsteckbar ist. Darüber hinaus weist das Stirnpolster (14) zur Bereitstellung einer ausreichenden Eigenstabilität innere Querstege (33) auf, die bei einer Ausbildung des Stirnpolsters (14) aus einem elastomeren Material zu einem guten Kompromiß zwischen einer ausreichenden Formstabilität und einer ausreichenden Nachgiebigkeit beitragen.

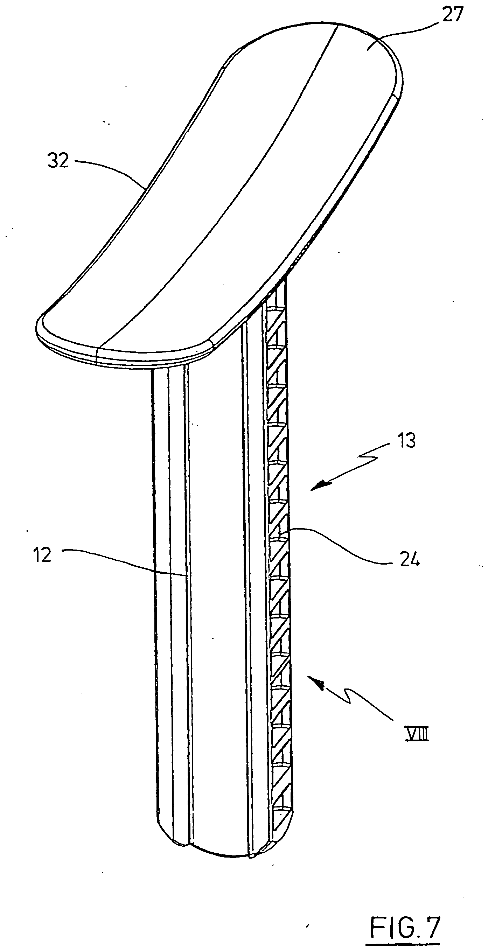

Die Darstellung des Stützelementes (13) in Fig. 7 veranschaulicht, daß durch die Rastung (24) eine Vielzahl von Rasterfächern ausgebildet werden, die jeweils zur Aufnahme der Rastvorsprünge (28) der Klemmstege (23) vorgesehen sind. Ebenfalls ist erkennbar, daß die Stützplatte (27) leicht gewölbt ist, um eine Anpassung an die Kontur einer menschlichen Stirn zu gewährleisten.

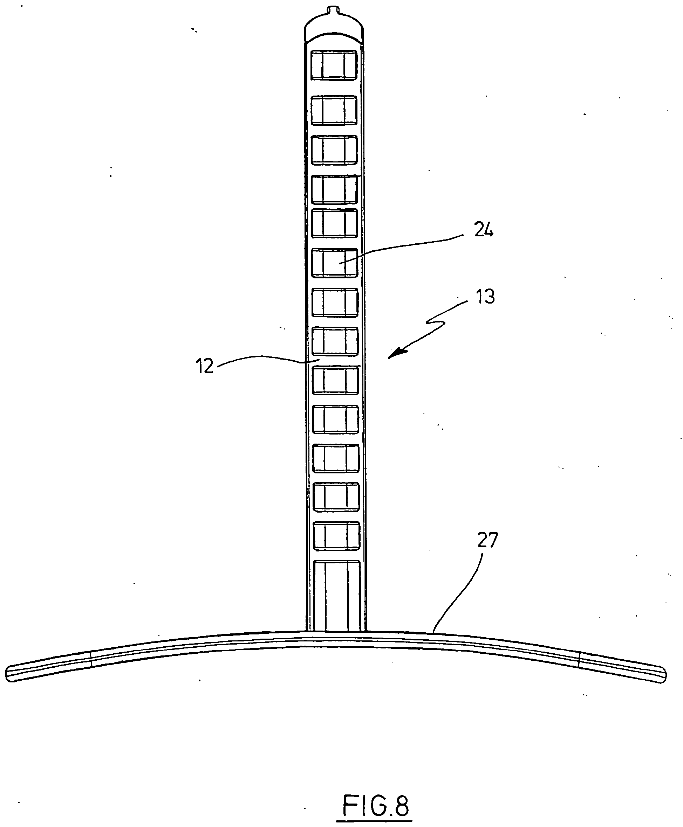

Die Wölbung der Stützplatte (27) sowie die Anordnung der Rastung (24) entlang der Längserstreckung des Schaftes (12) wird in Fig. 8 noch einmal veranschaulicht.

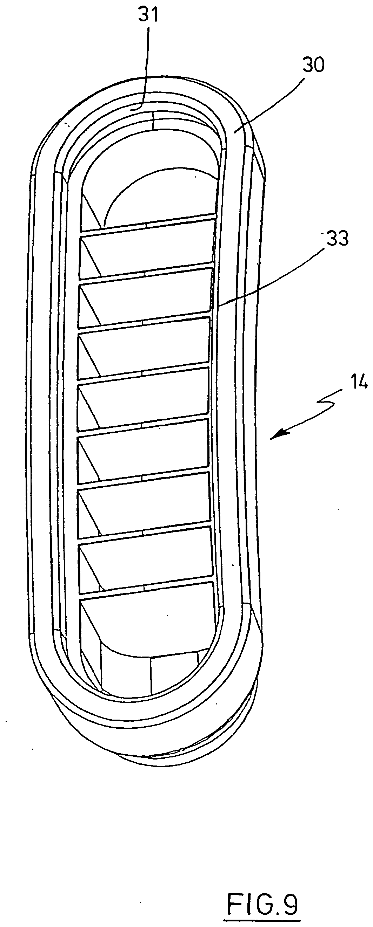

Aus der Darstellung in Fig. 9 ist der Aufbau des Stirnpolsters (14) in größerer Detailliertheit zu erkennen. Das Stirnpolster (14) weist eine haubenartige Querschnittgestaltung auf und von den Querstegen (33) werden sowohl Längsseiten des Stirnpolsters (14) relativ zueinander abgestützt als auch eine Versteifung im Bereich einer dem Rand (30) abgewandten Abschlußrundung hervorgerufen.

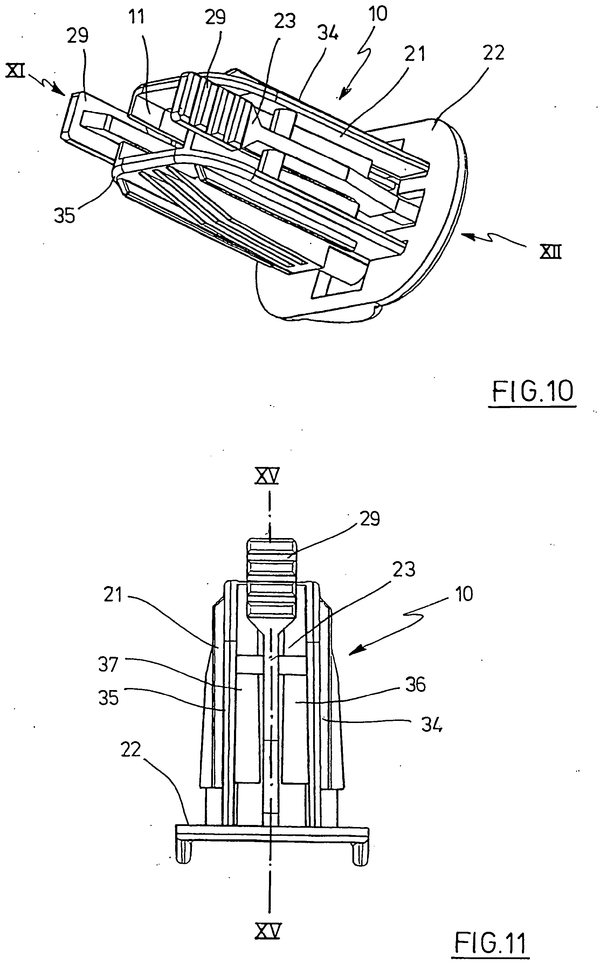

Die vergrößerte perspektivische Darstellung in Fig. 10 zeigt die Verstelleinrichtung (10) in stärkerer Detailliertheit. Es ist zu erkennen, daß der Grundkörper (21) im wesentlichen aus zwei Platten (34, 35) besteht, zwischen denen die Klemmstege (23) angeordnet sind.

Aus Fig. 11 ist zu erkennen, daß an den Platten (34, 35) Querstege (36, 37) angeordnet sind, die sich in Richtung auf die Klemmstege (23) erstrecken.

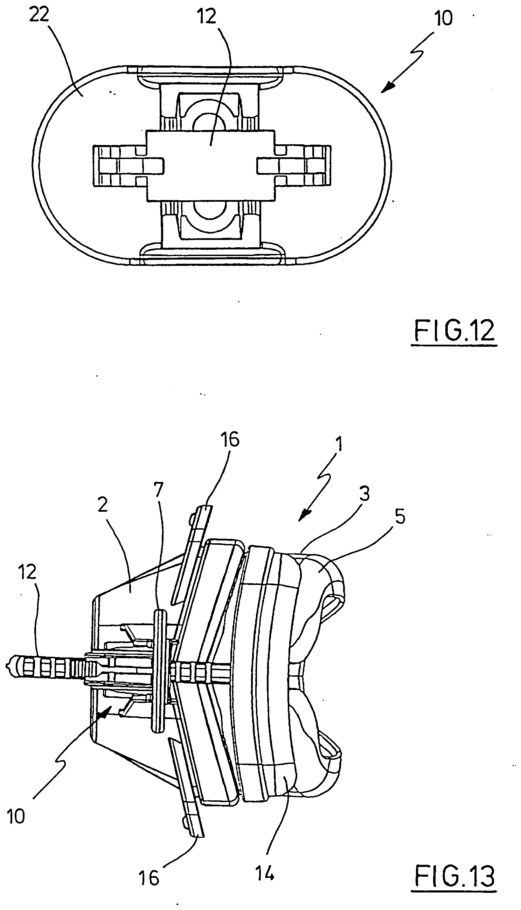

Fig. 12 veranschaulicht die Kombination der Verstelleinrichtung (10) mit dem Schaft (12). Fig. 13 zeigt in einer weiteren Seitenansicht als Zusammenbaudarstellung die Kombination der Atemmaske (1) mit einem Stirnpolster (14), das über einen Schaft (12) von einer Verstelleinrichtung (10) im Bereich des Halterungsschenkels (7) fixiert ist.

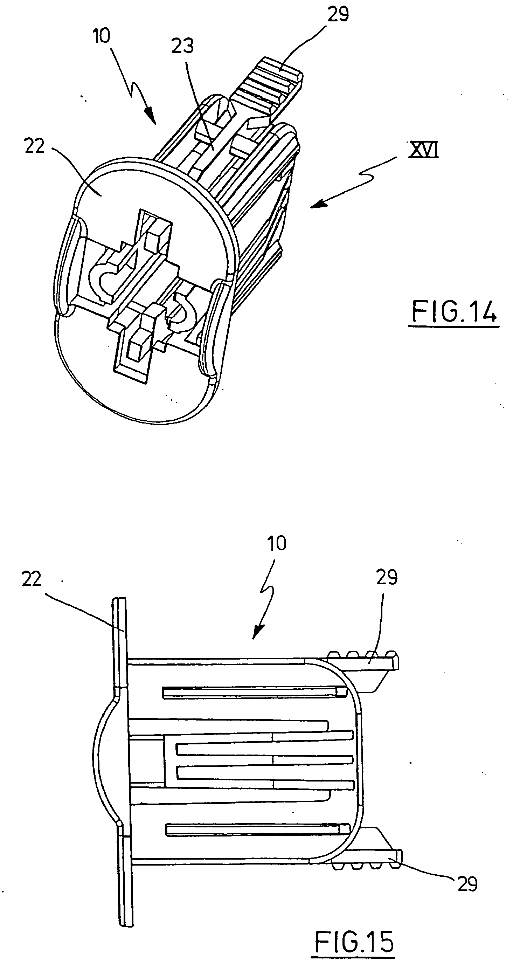

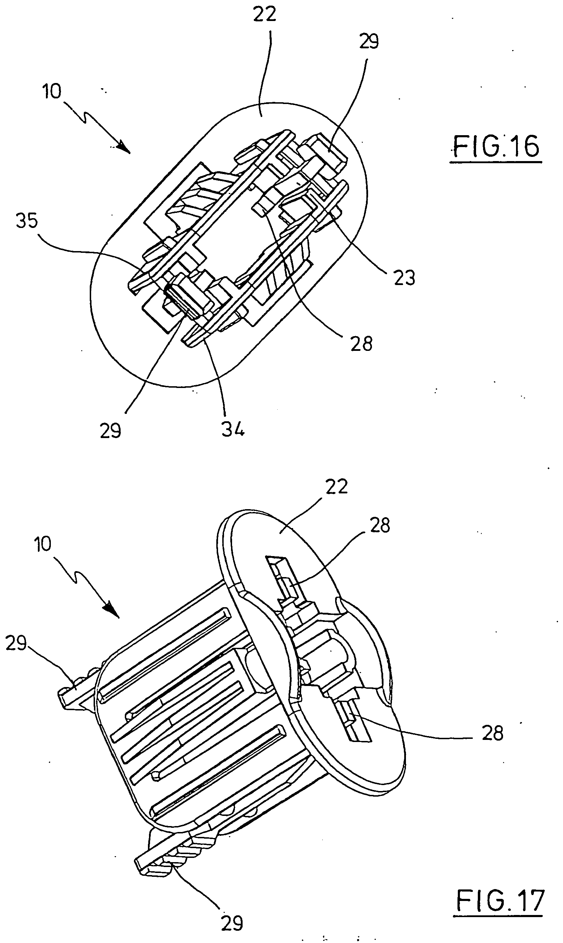

Fig. 14 und Fig. 15 zeigen nochmals den Aufbau der Verstelleinrichtung (10). Weitere Darstellungen der Verstelleinrichtung (10) finden sich auch in Fig. 16 und in Fig. 17.

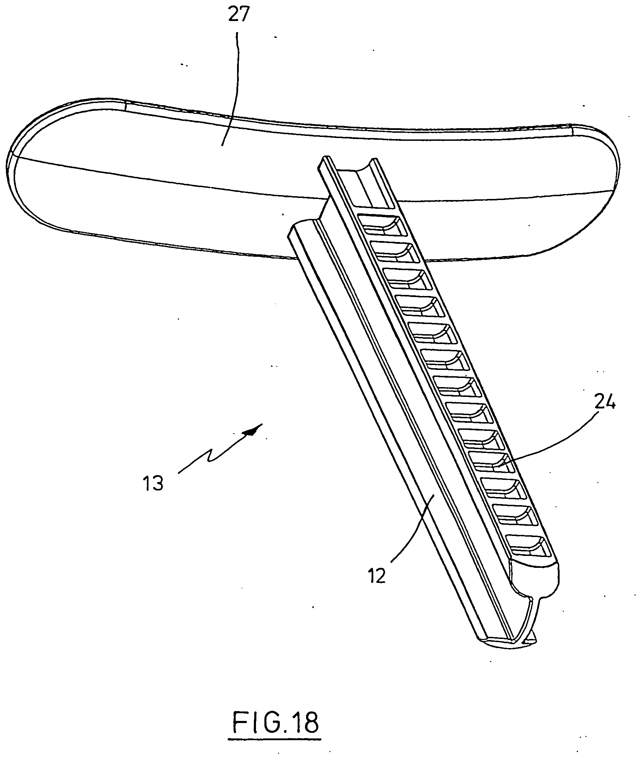

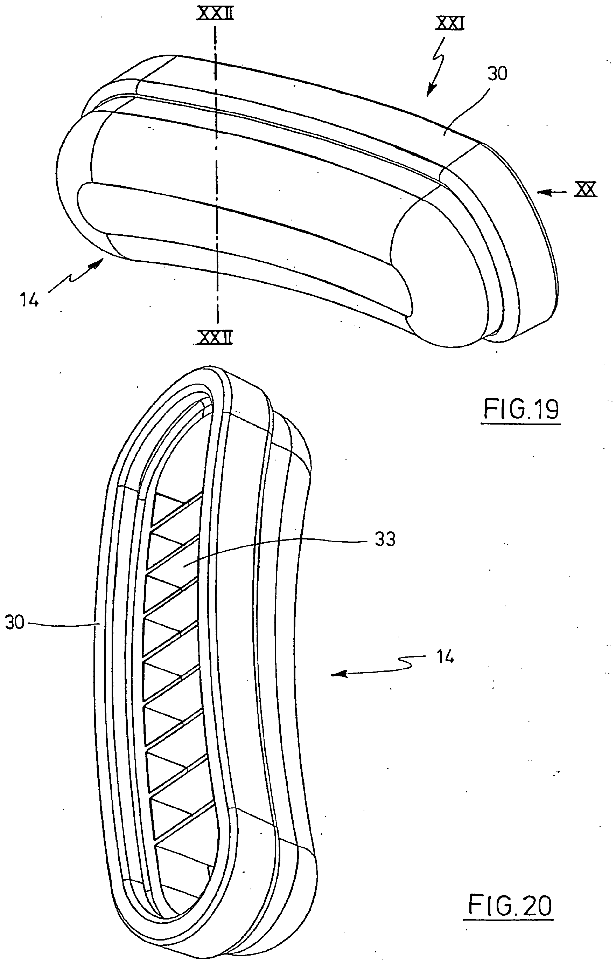

Fig. 18 zeigt in einer weiteren perspektivischen Darstellung nochmals das Stützelement (13), wobei insbesondere erkennbar ist, daß der Schaft (12) ein Querschnittprofil ähnlich zu einem I aufweist. Fig. 19 und Fig. 20 zeigen nochmals in weiteren perspektivischen Darstellungen die Gestaltung des Stirnpolsters (14).

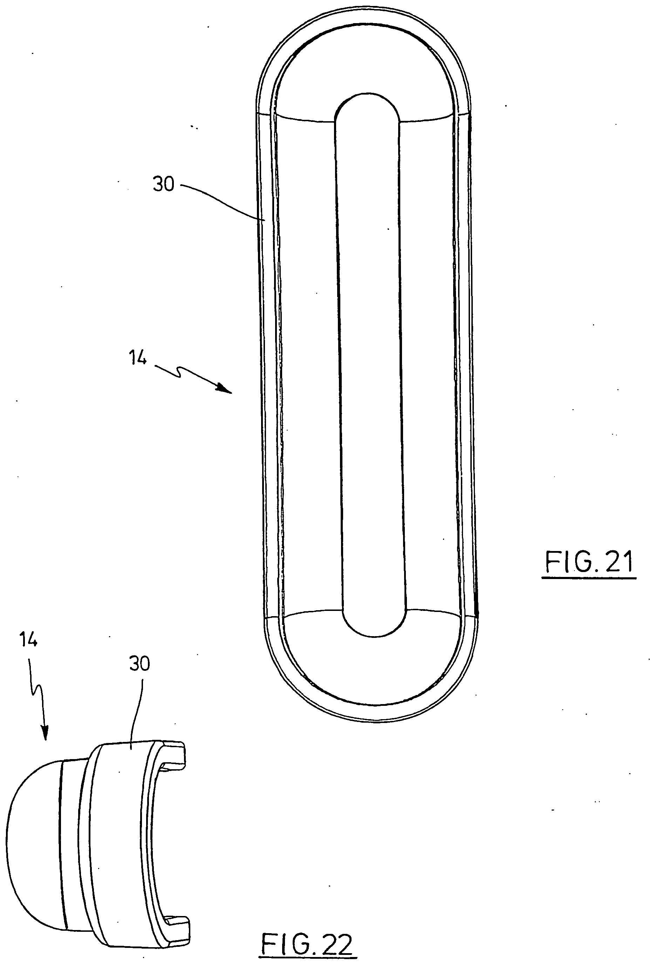

Fig. 21 veranschaulicht, daß das Stirnpolster (14) im wesentlichen eine symmetrische Gestaltung zu seiner Längsachse aufweist. Fig. 22 veranschaulicht die gerundete äußere Konturierung des Stirnpolsters (14).

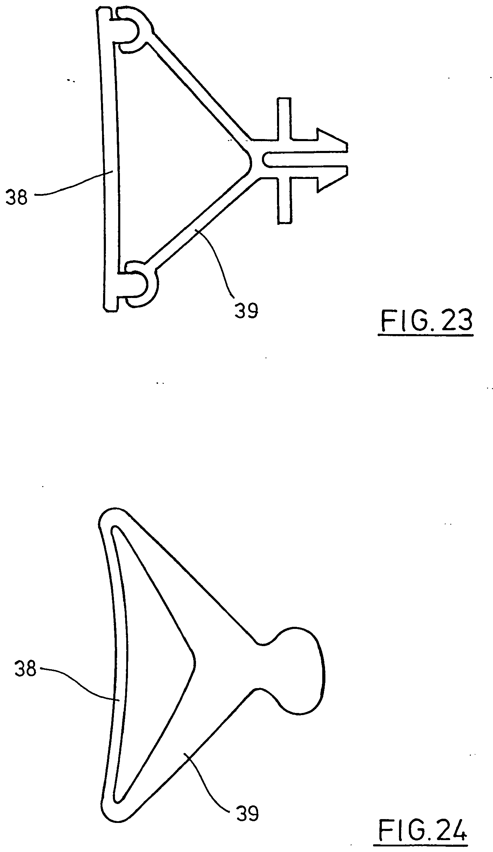

Fig. 23 zeigt eine modifizierte Ausführungsform, bei der ein elastomeres Band (38) von einer gabelartigen Halterung (39) positioniert ist. Das elastomere Band (38) kann sich der Stirnkontur und der Stirnneigung anpassen.

Fig. 24 zeigt eine zur Fig. 23 modifizierte Ausführungsform, bei der das elastomere Band (38) und die Halterung (39) einteilig ausgeführt sind. Als Werkstoff kann z.B. Schaum oder ein Hohlkörper verwendet werden.

Ein typischer Einstellvorgang erfolgt derart, daß der Patient zunächst die Atemmaske (1) im Bereich seiner Nase positioniert und in einem ersten Schritt eine Anpassung an die Stirnhöhe durch eine Verschiebung der Verstelleinrichtung (10) innerhalb der Montageöffnung (9) in Richtung der Längsachse (8) durchführt. Über die Rastung (15) wird eine definierte Positionierung unterstützt. Nach einer geeigneten Anordnung und Arretierung der Verstelleinrichtung (10) innerhalb der Montageöffnung (9) wird eine Anpassung an den Stirnabstand durch ein Herausziehen des Schaftes (12) aus der Führungsausnehmung (11) bzw. durch ein Hineinschieben des Schaftes (12) in die Führungsausnehmung (11) durchgeführt. Vor dieser Positionierung werden durch einen Druck auf die Bedienflächen (29) der Klemmstege (23) die Rastvorsprünge (28) aus den Vertiefungen der Rastung (24) herausgeschwenkt. Nach einer geeigneten Positionierung des Schaftes (12) des Stützelementes (13) werden die Bedienflächen (29) losgelassen und die Rastvorsprünge (28) federn in die Rastung (24) zurück. Hierdurch ist das Stützelement (13) in seiner aktuellen Positionierung arretiert.

Sollte eine nachträgliche Veränderung der gewählten Positionierungen erforderlich sein, so kann sowohl eine Änderung der Stirnhöheneinstellung als auch eine Änderung der Stirnabstandseinstellung ohne Rückwirkung auf die jeweils andere Einstellung erfolgen. Die erläuterten Arretierungen vermeiden eine ungewollte Veränderung der Positionierung des Stützelementes (13) während der Benutzung.

## Patentansprüche

1. Atemmaske (1) mit einer Vorrichtung zur Abstützung der Atemmaske (1) im Bereich einer Stirn eines Patienten, die mindestens ein im Bereich mindestens eines Halterungsschenkels (7) der Atemmaske (1) angeordnetes und über eine Verstelleinrichtung (10) mit dem Halterungsschenkel (7) verbundenes Stützelement (13) aufweist, sowie bei der der Halterungsschenkel (7) eine Längsachse (8) aufweist sowie relativ zu einem Maskengrundkörper (2) der Atemmaske (1) verschwenkbar angeordnet ist, und bei der das Stützelement (13) relativ zur Verstelleinrichtung (10) unbeweglich angeordnet ist, **dadurch gekennzeichnet, daß** die Verstelleinrichtung (10) eine Querführung für die Positionier rung des Stützelementes (13) im Wesentlichen quer zur Längsachse (8) des Halterungsschenkels (7) aufweist, wobei für das Stützelement (13) zur Fixierung einer Adaption an einen jeweiligen Stirnabstand eine lösbare Arretierung quer zur Längsachse (8) vorgesehen ist.

2. Vorrichtung nach Anspruch 1, **dadurch gekennzeichnet, daß** die Verstelleinrichtung (10) eine Längsführung für das Stützelement (13) in Richtung der Längsachse (8) des Halterungsschenkels (7) aufweist, wobei für die Verstelleinrichtung (10) zur Fixierung einer Adaption an eine jeweilige Stirnhöhe eine Arretierung in Richtung der Längsachse vorgesehen ist.

3. Vorrichtung nach Anspruch 2, **dadurch gekennzeichnet, daß** für die Führung der Verstelleinrichtung (10) in Richtung der Längsachse (8) eine Rastung vorgesehen ist.

4. Vorrichtung nach Anspruch 2, **dadurch gekennzeichnet, daß** dief Verstelleinrichtung (10) in Richtung der Längsachse (8) stufenlos verstellbar ist.

5. Vorrichtung nach einem der Ansprüche 1 bis 4, **dadurch gekennzeichnet, daß** für die Verstellung des Stützelementes (13) quer zur Längsachse (8) eine Rastung vorgesehen ist.

6. Vorrichtung nach einem der Ansprüche 1 bis 4, **dadurch gekennzeichnet, daß** das Stützelement (13) quer zur Längsachse (8) stufenlos verstellbar ist.

7. Vorrichtung nach einem der Ansprüche 1 bis 6, **dadurch gekennzeichnet, daß** das Stützelement (13) ein Stirnpolster (14) trägt, das als ein Gelkissen ausgebildet ist.

8. Vorrichtung nach einem der Ansprüche 1 bis 6, **dadurch gekennzeichnet, daß** das Stützelement (13) ein Stirnpolster (14) trägt, das als ein elastomeres Polster ausgebildet ist.

9. Vorrichtung nach einem der Ansprüche 1 bis 6, **dadurch gekennzeichnet, daß** das Stützelement (13) ein Stirnpolster (14) trägt, das als ein Schaumpolster ausgebildet ist.

10. Vorrichtung nach einem der Ansprüche 1 bis 9, **dadurch gekennzeichnet, daß** das Stützelement (13) relativ zum Halterungsschenkel (7) verschwenkbar angeordnet ist.

11. Vorrichtung nach einem der Ansprüche 7 bis 9, **dadurch gekennzeichnet, daß** das Stirnpolster (13) in frontaler Richtung verkippbar angeordnet ist.

12. Vorrichtung nach einem der Ansprüche 7 bis 9, **dadurch gekennzeichnet, daß** das Stirnpolster (14) seitlich kippbar angeordnet ist.

## Claims

1. Respiratory mask (1) having a device for supporting the respiratory mask (1) in the region of a forehead of a patient, said device having at least one support element (13) which is disposed in the region of at least one mounting leg (7) of the respiratory mask (1) and which is connected to the mounting leg (7) by way of an adjustment installation (10), and in which respiratory mask (1) the mounting leg (7) has a longitudinal axis (8) and is disposed so as to be pivotable relative to a mask main body (2) of the respiratory mask (1), and in which the support element (13) is disposed so as to be immovable relative to the adjustment installation (10), **characterized in that**
the adjustment installation (10) has a transverse guide for positioning the support element (13) in a manner substantially transverse to the longitudinal axis (8) of the mounting leg (7), wherein a releasable locking mechanism that is transverse to the longitudinal axis (8) is provided for the support element (13) in order for a customization to a respective forehead spacing to be fixed.

2. Device according to Claim 1, **characterized in that** the adjustment installation (10) has a longitudinal guide for the support element (13) in the direction of the longitudinal axis (8) of the mounting leg (7), wherein a locking mechanism in the direction of the longitudinal axis is provided for the adjustment installation (10) in order for a customization to a respective forehead height to be fixed.

3. Device according to Claim 2, **characterized in that** a latching mechanism is provided for the guide of the adjustment installation (10) in the direction of the longitudinal axis (8).

4. Device according to Claim 2, **characterized in that** the adjustment installation (10) is adjustable in a stepless manner in the direction of the longitudinal axis (8).

5. Device according to one of Claims 1 to 4, **characterized in that** a latching mechanism is provided for the adjustment of the support element (13) transversely to the longitudinal axis (8).

6. Device according to one of Claims 1 to 4, **characterized in that** the support element (13) is adjustable in a stepless manner transversely to the longitudinal axis (8).

7. Device according to one of Claims 1 to 6, **characterized in that** the support element (13) supports a forehead cushion (14) which is configured as a gel pad.

8. Device according to one of Claims 1 to 6, **characterized in that** the support element (13) supports a forehead cushion (14) which is configured as an elastomeric cushion.

9. Device according to one of Claims 1 to 6, **characterized in that** the support element (13) supports a forehead cushion (14) which is configured as a foam cushion.

10. Device according to one of Claims 1 to 9, **characterized in that** the support element (13) is disposed so as to be pivotable relative to the mounting leg (7).

11. Device according to one of Claims 7 to 9, **characterized in that** the forehead cushion (13) is disposed so as to be capable of being tilted in the frontal direction.

12. Device according to one of Claims 7 to 9, **characterized in that** the forehead cushion (14) is disposed so as to be capable of being laterally tilted.

## Revendications

1. Masque respiratoire (1) avec un dispositif pour supporter le masque respiratoire (1) dans la région du front d'un patient, qui présente au moins un élément de support (13) disposé dans la région d'au moins une branche de fixation (7) du masque respiratoire (1) et connecté à la branche de fixation (7) par le biais d'un dispositif de réglage (10), et dans lequel la branche de fixation (7) présente un axe longitudinal (8) et est disposé de manière à pouvoir pivoter par rapport à un corps de base de masque (2) du masque respiratoire (1),
et dans lequel l'élément de support (13) est disposé de manière immobile par rapport au dispositif de réglage (10), **caractérisé en ce que** le dispositif de réglage (10) présente un guide transversal pour le positionnement de l'élément de support (13) essentiellement transversalement par rapport à l'axe longitudinal (8) de la branche de fixation (7), un verrouillage desserrable étant prévu transversalement à l'axe longitudinal (8) pour l'élément de support (13) afin de fixer une adaptation à une distance respective du front.

2. Dispositif selon la revendication 1, **caractérisé en ce que** le dispositif de réglage (10) présente un guidage longitudinal pour l'élément de support (13) dans la direction de l'axe longitudinal (8) de la branche de fixation (7), un verrouillage dans la direction de l'axe longitudinal étant prévu pour le dispositif de réglage (10) afin de fixer une adaptation à une hauteur respective du front.

3. Dispositif selon la revendication 2, **caractérisé en ce qu'**un crantage est prévu pour le guidage du dispositif de réglage (10) dans la direction de l'axe longitudinal (8).

4. Dispositif selon la revendication 2, **caractérisé en ce que** le dispositif de réglage (10) peut être réglé en continu dans la direction de l'axe longitudinal (8).

5. Dispositif selon l'une quelconque des revendications 1 à 4, **caractérisé en ce qu'**un crantage est prévu pour le réglage de l'élément de support (13) transversalement à l'axe longitudinal (8) .

6. Dispositif selon l'une quelconque des revendications 1 à 4, **caractérisé en ce que** l'élément de support (13) peut être réglé en continu transversalement à l'axe longitudinal (8).

7. Dispositif selon l'une quelconque des revendications 1 à 6, **caractérisé en ce que** l'élément de support (13) porte un rembourrage pour le front (14), qui est réalisé sous la forme d'un coussin de gel.

8. Dispositif selon l'une quelconque des revendications 1 à 6, **caractérisé en ce que** l'élément de support (13) supporte un rembourrage pour le front (14) qui est réalisé sous la forme d'un rembourrage en élastomère.

9. Dispositif selon l'une quelconque des revendications 1 à 6, **caractérisé en ce que** l'élément de support (13) porte un rembourrage pour le front (14) qui est réalisé sous la forme d'un rembourrage en mousse.

10. Dispositif selon l'une quelconque des revendications 1 à 9, **caractérisé en ce que** l'élément de support (13) est disposé de manière à pouvoir pivoter par rapport à la branche de fixation (7).

11. Dispositif selon l'une quelconque des revendications 7 à 9, **caractérisé en ce que** le rembourrage pour le front (13) est disposé de manière à pouvoir basculer dans la direction frontale.

12. Dispositif selon l'une quelconque des revendications 7 à 9, **caractérisé en ce que** le rembourrage pour le front (14) est disposé de manière à pouvoir basculer latéralement.
